# EUROPÄISCHE PATENTANMELDUNG

(11) **EP 3 550 241 A1**
(43) Veröffentlichungstag der Anmeldung: **09.10.2019**
(21) Anmeldenummer: 18166169.5
(22) Anmeldetag: 06.04.2018
(51) Int. Cl.: F25J 3/02, C10G 70/04

(54) **VERFAHREN UND EINE ANLAGE ZUR TRENNUNG EINES KOHLENWASSERSTOFFGEMISCHS**

(71) Anmelder: Linde AG, 80331 München (DE)
(72) Erfinder: Duc, Tuat Pham, 82377 Penzberg (DE); Kurz, Benedikt, 80331 München (DE)
(74) Vertreter: m patent group

(57) **Zusammenfassung**

Die Erfindung betrifft ein Verfahren zur Trennung eines Komponentengemischs (K), das Wasserstoff, Methan, Kohlenwasserstoffe mit zwei Kohlenstoffatomen und Kohlenwasserstoffe mit drei und mehr Kohlenstoffatomen enthält, wobei zunächst wenigstens 95% der Kohlenwasserstoffe mit drei und mehr Kohlenstoffatomen aus zumindest einem Teil des Gasgemischs abgetrennt werden und anschließend aus dem verbleibenden Rest wenigstens 95% des Methans und des Wasserstoffs abgetrennt werden. Es ist vorgesehen, dass zumindest ein Teil des Komponentengemischs (K) durch Abkühlen von einem ersten Temperaturniveau auf ein zweites Temperaturniveau auf einem ersten Druckniveau unter Erhalt eines ersten Gasfraktion (G1) und einer ersten Flüssigfraktion (C1) einer ersten Teilkondensation unterworfen wird, zumindest ein Teil der ersten Gasfraktion (G1) durch Abkühlen von dem zweiten Temperaturniveau auf ein drittes Temperaturniveau auf dem ersten Druckniveau unter Erhalt einer zweiten Gasfraktion (G4) und einer zweiten Flüssigfraktion (C2) einer zweiten Teilkondensation unterworfen wird, zumindest ein Teil der ersten Flüssigfraktion (C1) und der zweiten Flüssigfraktion (C1) von dem ersten Druckniveau auf ein zweites Druckniveau entspannt und unter Erhalt einer dritten Gasfraktion (G3) und einer dritten Flüssigfraktion (C3+) einer Rektifikation unterworfen werden, die zweite Gasfraktion (G4) und die dritte Gasfraktion (G3) derart gebildet werden, dass sie mehr als 95% Wasserstoff, Methan und Kohlenwasserstoffe mit zwei Kohlenstoffatomen aufweisen, und die dritte Flüssigfraktion (C3+) derart gebildet wird, dass sie mehr als 95% Kohlenwasserstoffe mit drei und mehr Kohlenstoffatomen aufweist. Eine entsprechende Anlage (100, 200) ist ebenfalls Gegenstand der vorliegenden Erfindung.

## Beschreibung

Die Erfindung betrifft ein Verfahren und eine Anlage zur Trennung eines Komponentengemischs gemäß den Oberbegriffen der unabhängigen Patentansprüche.

### Stand der Technik

Verfahren und Vorrichtungen zum Dampfspalten (engl. Steam Cracking) von Kohlenwasserstoffen sind bekannt und beispielsweise im Artikel "Ethylene" in Ullmann's Encyclopedia of Industrial Chemistry, online seit 15. April 2007, DOI 10.1002/14356007.a10_045.pub2, beschrieben.

Durch Dampfspalten, aber auch unter Verwendung anderer Verfahren und Vorrichtungen, werden Kohlenwasserstoffgemische enthalten. Diese müssen zumindest teilweise in die jeweils enthaltenen Komponenten aufgetrennt werden. Dies kann in Trennsequenzen unterschiedlicher Art erfolgen. Entsprechende Trennsequenzen umfassen, nach vorheriger Abtrennung von Wasser, schweren Komponenten und Sauergasen, Einrichtungen zur Demethanisierung, Deethanisierung und Depropanisierung (engl. Demethanizer, Deethanizer, Depropanizer, siehe insbesondere Abschnitt 5.3.2.2, "Hydrocarbon Fractionation Section" in dem erwähnten Ullmann-Artikel). Die Reihenfolge von Demethanisierung, Deethanisierung und Depropanisierung kann dabei variieren.

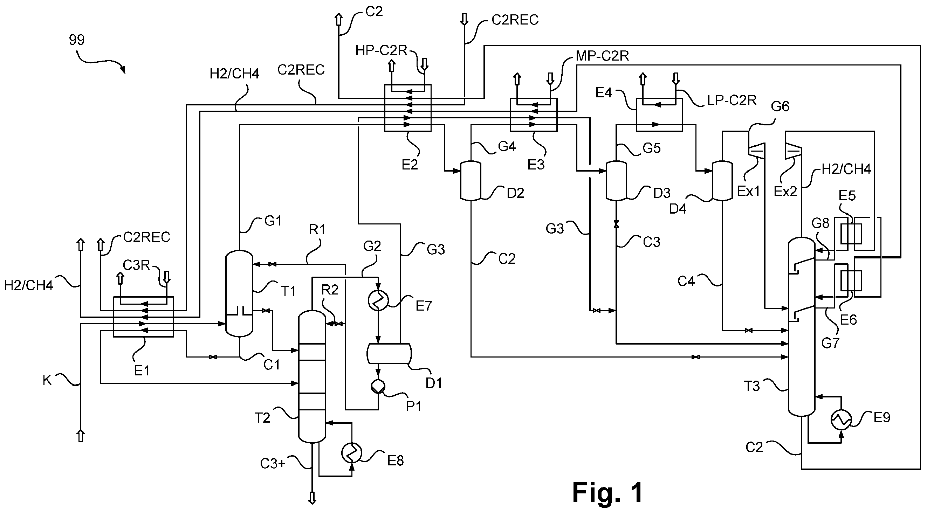

Ein Verfahren bzw. eine Anlage zur Trennung eines entsprechendes Gasgemischs, in dem bzw. der eine überwiegend oder ausschließlich Methan und Wasserstoff enthaltende erste Fraktion (C1 minus-Fraktion), eine überwiegend oder ausschließlich Kohlenwasserstoffe mit zwei Kohlenstoffatomen enthaltende Fraktion (C2-Fraktion) und eine überwiegend oder ausschließlich Kohlenwasserstoffe mit drei und mehr Kohlenstoffatomen enthaltende Fraktion (C3plus-Fraktion) gebildet werden, ist in Figur 1 veranschaulicht und wird unter Bezugnahme auf diese Figur unten erläutert. Hierbei folgt eine Demethanisierung auf eine Deethanisierung, wobei für beide Verfahrensschritte Rektifikationskolonnen verwendet werden.

Vorteile dieses Verfahrens bzw. dieser Anlage sind eine einfache Prozessführung und eine hohe Energieeffizienz. Nachteilig sind hingegen die Komplexität der Deethanisierung mit einem zusätzlichen sogenannten C3-Absorber und die Komplexität der Demethanisierung mit Zwischenkühler und Kopfkondensator, die oberhalb der Demethanisierungskolonne angeordneten sind. Letztere erfordern einen erhöhten Instrumentierungs- und Sicherheitsaufwand.

Die vorliegende Erfindung stellt sich daher die Aufgabe, verbesserte Maßnahmen zur Trennung entsprechender Gasgemische anzugeben.

### Offenbarung der Erfindung

Die Erfindung schlägt vor diesem Hintergrund ein Verfahren und eine Anlage zur Trennung eines Komponentengemischs mit den jeweiligen Merkmalen der unabhängigen Patentansprüche vor. Bevorzugte Ausgestaltungen sind Gegenstand der abhängigen Patentansprüche und der nachfolgenden Beschreibung.

Vor der Erläuterung der Merkmale und Vorteile der vorliegenden Erfindung werden deren Grundlagen und die verwendeten Begriffe erläutert.

Die vorliegende Erfindung kommt zur Trennung von Komponentengemischen zum Einsatz, welche überwiegend oder ausschließlich Wasserstoff, Methan, Kohlenwasserstoffe mit zwei Kohlenstoffatomen (Ethan, Ethylen und ggf. Acetylen, falls nicht bereits in einer zuvor erfolgenden Hydrierung umgesetzt), und Kohlenwasserstoffe mit drei und mehr Kohlenstoffatomen (Propan, Propylen, ggf. Methylacetylen und schwerere Kohlenwasserstoffe mit insbesondere vier, fünf, sechs und mehr Kohlenstoffatomen) enthalten.

Im Rahmen der vorliegenden Erfindung werden entsprechende Komponentengemische insbesondere unter Einsatz eines Dampfspaltverfahrens gebildet. In dem Dampfspaltverfahren wird dabei ein sogenanntes Rohgas bzw. Spaltgas erhalten, das neben den erwähnten Komponenten auch weitere Komponenten aufweist. Diese, insbesondere Wasser, Sauergase wie Kohlendioxid und Schwefelwasserstoff sowie benzin- und ölartige Komponenten, können stromauf des erfindungsgemäß vorgeschlagenen Verfahrens bzw. einer entsprechenden Anlage abgetrennt werden. Auch weitere Verfahrensschritte können stromauf des erfindungsgemäß vorgeschlagenen Verfahrens bzw. einer entsprechenden Anlage durchgeführt werden, insbesondere eine Hydrierung von Acetylenen (sogenannte Front-End-Hydrierung). Das im Rahmen der vorliegenden Erfindung bearbeitete Komponentengemisch liegt in verdichtetem Zustand und typischerweise bei Umgebungstemperatur vor.

In der Fachwelt werden für Fraktionen, die in entsprechenden Verfahren aus den genannten Komponentengemischen gebildet werden, Kurzbezeichnungen verwendet, die die Kohlenstoffanzahl der jeweils überwiegend oder ausschließlich enthaltenen Kohlenwasserstoffe angeben. So ist eine "C1-Fraktion" eine Fraktion, die überwiegend oder ausschließlich Methan (jedoch konventionsgemäß unter Umständen auch Wasserstoff, dann auch "C1 minus-Fraktion" genannt) enthält. Eine "C2-Fraktion" enthält hingegen überwiegend oder ausschließlich Ethan, Ethylen und/oder Acetylen. Eine "C3-Fraktion" enthält überwiegend Propan, Propylen, Methylacetylen und/oder Propadien. Entsprechendes gilt für eine "C4-Fraktion" und die höheren Fraktionen. Mehrere Fraktionen können auch verfahrens- und/oder bezeichnungsmäßig zusammengefasst werden. Beispielsweise enthält eine "C2plus-Fraktion" überwiegend oder ausschließlich Kohlenwasserstoffe mit zwei und mehr und eine "C2minus-Fraktion" überwiegend oder ausschließlich Kohlenwasserstoffe mit einem oder zwei Kohlenstoffatomen und ggf. Wasserstoff.

Komponentengemische können im hier verwendeten Sprachgebrauch reich oder arm an einer oder mehreren Komponenten sein, wobei "reich" für einen Gehalt von wenigstens 90%, 95%, 99%, 99,5%, 99,9%, 99,99% oder 99,999% und "arm" für einen Gehalt von höchstens 10%, 5%, 1%, 0,1%, 0,01% oder 0,001% auf molarer, Gewichts- oder Volumenbasis stehen kann. Komponentengemische können im hier verwendeten Sprachgebrauch ferner angereichert oder abgereichert an einer odermehreren Komponenten sein, wobei sich diese Begriffe auf einen entsprechenden Gehalt in einem anderen Komponentengemisch (Ausgangsgemisch) beziehen, aus dem das Komponentengemisch erhalten wurde. Das Komponentengemisch ist "angereichert", wenn dieses zumindest den 1,1-fachen, 1,5-fachen, 2-fachen, 5-fachen, 10-fachen, 100-fachen oder 1.000-fachen Gehalt, "abgereichert" wenn es höchstens den 0,9-fachen, 0,5-fachen, 0,1-fachen, 0,01-fachen oder 0,001-fachen Gehalt einer entsprechenden Komponente bezogen auf das Ausgangsgemisch, enthält. Ist hier davon die Rede, dass ein Komponentengemisch "im Wesentlichen" oder "zumindest überwiegend" eine oder mehrere Komponenten enthält, wird hierunter insbesondere verstanden, dass das Komponentengemisch zumindest reich an der einen oder den mehreren Komponenten im oben erläuterten Sinn ist oder ausschließlich die eine oder die mehreren Komponenten aufweist.

Ein Komponentengemisch ist von einem Ausgangsgemisch "abgeleitet" oder aus diesem "gebildet", wenn es zumindest einige in dem Ausgangsgemisch enthaltene oder aus diesem erhaltene Komponenten aufweist. Ein in diesem Sinne abgeleitetes oder gebildetes Komponentengemisch kann dabei aus dem Ausgangsgemisch durch Abtrennen oder Abzweigen eines Teilstroms oder einer oder mehrerer Komponenten, Anreichern oder Abreichern bezüglich einer oder mehrerer Komponenten, chemisches oder physikalisches Umsetzen einer oder mehrerer Komponenten, Erwärmen, Abkühlen, Druckbeaufschlagen und dergleichen erhalten bzw. gebildet werden.

Die vorliegende Anmeldung verwendet zur Charakterisierung von Drücken und Temperaturen die Begriffe "Druckniveau" und "Temperaturniveau", wodurch zum Ausdruck gebracht werden soll, dass entsprechende Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, die beispielsweise ± 1%, 5%, 10%, 20% oder sogar 50% um einen Mittelwert liegen. Entsprechende Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste, beispielsweise aufgrund von Abkühlungseffekten, ein. Entsprechendes gilt für Temperaturniveaus. Bei den hier in bar angegebenen Druckniveaus handelt es sich um Absolutdrücke.

Ein "Wärmetauscher" dient zur indirekten Übertragung von Wärme zwischen zumindest zwei z.B. im Gegenstrom zueinander geführten Strömen, beispielsweise einem wärmeren gasförmigen Druckstrom und einem oder mehreren kälteren flüssigen Strömen. Ein Wärmetauscher kann aus einem einzelnen oder mehreren parallel und/oder seriell verbundenen Wärmetauscherabschnitten gebildet sein, z.B. aus einem oder mehreren Plattenwärmetauscherblöcken. Ein Wärmetauscher weist "Passagen" auf, die als getrennte Fluidkanäle mit Wärmeaustauschflächen ausgebildet sind.

Bei einer "Rektifikationskolonne" handelt es sich im hier verwendeten Sprachgebrauch um eine Trenneinheit, die dafür eingerichtet ist, ein gasförmig oder flüssig oder in Form eines Zweiphasengemisch mit flüssigen und gasförmigen Anteilen, ggf. auch im überkritischen Zustand, bereitgestelltes Stoffgemisch (Fluid) zumindest teilweise aufzutrennen, also aus dem Stoffgemisch jeweils Reinstoffe oder Stoffgemische zu erzeugen, die gegenüber dem Stoffgemisch bezüglich zumindest einer Komponente angereichert bzw. abgereichert oder reich bzw. arm im oben erläuterten Sinne sind. Rektifikationskolonnen sind aus dem Bereich der Trenntechnik hinlänglich bekannt. Typischerweise sind Rektifikationskolonnen als zylindrische Metallbehälter ausgebildet, die mit Einbauten, beispielsweise Siebböden oder geordneten und ungeordneten Packungen, ausgerüstet sind. Eine Rektifikationskolonne zeichnet sich unter anderem dadurch aus, dass sich in ihrem unteren Bereich, auch als Sumpf bezeichnet, eine flüssige Fraktion abscheidet. Diese flüssige Fraktion, die hier als Sumpfflüssigkeit bezeichnet wird, wird in einer Rektifikationskolonne mittels eines Sumpfverdampfers erwärmt, so dass kontinuierlich ein Teil der Sumpfflüssigkeit verdampft und in der Rektifikationskolonne gasförmig aufsteigt. Eine Rektifikationskolonne ist ferner mit einem sogenannten Kopfkondensator versehen, in den zumindest ein Teil eines sich in einem oberen Bereich der Rektifikationskolonne anreichernden Gasgemischs oder ein entsprechendes Reingas, hier als Kopfgas bezeichnet, eingespeist, zu einem Teil zu einem Kondensat verflüssigt und als flüssiger Rücklauf am Kopf der Rektifikationskolonne aufgegeben wird. Ein Teil des aus dem Kopfgas enthaltenen Kondensats kann anderweitig verwendet werden.

Eine "Stripkolonne" unterscheidet sich von einer Rektifikationskolonne im Wesentlichen durch das Fehlen eines Kopfkondensators und die fehlende Verwendung eines aus Kopfgas gebildeten Rücklaufs zur Trennung. In eine Stripkolonne können jedoch in unterschiedlichen Höhen Flüssigkeiten eingespeist werden, die einen gewissen Rücklauf liefern bzw. mittels derer eine in der Stripkolonne herablaufende Flüssigfraktion bereitgestellt wird, die mit einer Gasphase im Austausch steht. Jedoch ist eine Stripkolonne, wie sie im Rahmen der vorliegenden Erfindung zum Einsatz kommt, ein ohne eigenes Kopfgaskondensat betriebener Apparat.

Zur Auslegung und spezifischen Ausgestaltung von Rektifikationskolonnen und anderen Trennkolonnen sei auf einschlägige Lehrbücher verwiesen (siehe beispielsweise Sattler, K: Thermische Trennverfahren: Grundlagen, Auslegung, Apparate, 3. Auflage 2001, Weinheim; Wiley-VCH).

### Vorteile der Erfindung

Die vorliegende Erfindung schlägt insgesamt ein Verfahren zur Trennung eines Komponentengemischs, das Wasserstoff, Methan, Kohlenwasserstoffe mit zwei Kohlenstoffatomen und Kohlenwasserstoffe mit drei und mehr Kohlenstoffatomen enthält, vor. Bei dem Verfahren werden, wie insoweit bekannt, zunächst wenigstens 95%, 96%, 97%, 98% oder 99% der Kohlenwasserstoffe mit drei und mehr Kohlenstoffatomen aus zumindest einem Teil des Gasgemischs abgetrennt und anschließend aus dem verbleibenden Rest wenigstens 95%, 96%, 97%, 98% oder 99% des Methans und des Wasserstoffs abgetrennt. Die vorliegende Erfindung betrifft also ein sogenanntes Deethanizer-First- bzw. Frontend-Deethanizer-Verfahren, wie es grundsätzlich aus dem Stand der Technik bekannt ist.

Erfindungsgemäß wird dabei zumindest ein Teil des Komponentengemischs durch Abkühlen von einem ersten Temperaturniveau auf ein zweites Temperaturniveau auf einem ersten Druckniveau unter Erhalt eines ersten Gasfraktion und einer ersten Flüssigfraktion einer ersten Teilkondensation unterworfen. Die erste
Gasfraktion und die erste Flüssigfraktion werden dabei im Rahmen der Teilkondensation rein kondensativ gebildet. Unter einer "rein kondensativen" Bildung sei dabei insbesondere verstanden, dass bei der Bildung der ersten Gasfraktion und der ersten Flüssigfraktion kein Rücklauf, also keine Absorptionsflüssigkeit zum Auswaschen bestimmter Komponenten zum Einsatz kommt. Eine Gesamtmenge der in einem bestimmten Zeitraum gebildeten ersten Gasfraktion und der in demselben Zeitraum gebildeten ersten Flüssigfraktion entspricht also im Rahmen der vorliegenden Erfindung der Menge des Komponentengemischs, das zur Bildung der ersten Gasfraktion und der ersten Flüssigfraktion verwendet wird. In diesem Punkt unterscheidet sich das erfindungsgemäße Verfahren von Verfahren zur Deethanisierung gemäß dem Stand der Technik, in denen sogenannte C3-Absorber zum Einsatz kommen. In einem C3-Absorber erfolgt ebenfalls eine Bildung einer ersten Gasfraktion und einer ersten Flüssigfraktion, aber unter Zuspeisung eines Rücklaufs, um aus der Gasphase nicht vollständig kondensierte Kohlenwasserstoffe mit drei Kohlenstoffatomen auszuwaschen.

Das der Abkühlung von dem ersten Temperaturniveau auf das zweite Temperaturniveau auf dem ersten Druckniveau unterworfene Komponentengemisch weist insbesondere 32 bis 36 Molprozent Wasserstoff, 5 bis 8 Molprozent Methan, bis 57 Molprozent Kohlenwasserstoffe mit zwei Kohlenstoffatomen und bis 4 Molprozent Kohlenwasserstoffe mit drei und mehr Kohlenstoffatomen auf. Die vorliegende Erfindung eignet sich damit besonders für solche Gasgemische, die aus Verfahren zum Dampfspalten von gasförmigen Einsätzen, stammen. Ein gasförmiger Einsatz umfasst dabei überwiegend oder ausschließlich Ethan oder Ethan und Propan. Die in der ersten Teilkondensation gebildete erste Gasfraktion weist insbesondere 43 bis 47 Molprozent Wasserstoff, 7 bis 9 Molprozent Methan, 42 bis 45 Molprozent Kohlenwasserstoffe mit zwei Kohlenstoffatomen und 0,5 bis 0,7 Molprozent Kohlenwasserstoffe mit drei und mehr Kohlenstoffatomen auf. Die in der ersten Teilkondensation gebildete erste Flüssigfraktion weist insbesondere 1 bis 2 Molprozent Wasserstoff, 2 bis 3 Molprozent Methan, 82 bis 85 Molprozent Kohlenwasserstoffe mit zwei Kohlenstoffatomen und 10 bis 13 Molprozent Kohlenwasserstoffe mit drei und mehr Kohlenstoffatomen auf. Mit anderen Worten enthält die erste Gasfraktion noch nennenswerte Mengen an Kohlenwasserstoffen mit drei und mehr Kohlenstoffatomen, die zurückzugewinnen sind. Dies erfolgt im Rahmen der vorliegenden Erfindung wie nachfolgend erläutert.

Im Rahmen der vorliegenden Erfindung wird zumindest ein Teil der ersten Gasfraktion durch Abkühlen von dem zweiten Temperaturniveau auf ein drittes Temperaturniveau auf dem ersten Druckniveau unter Erhalt einer zweiten Gasfraktion und einer zweiten Flüssigfraktion einer zweiten Teilkondensation unterworfen. Im Rahmen der zweiten Teilkondensation werden die zuvor in der ersten Gasfraktion enthaltenen Kohlenwasserstoffe mit drei und mehr Kohlenstoffatomen bis auf etwaige Restgehalte abgeschieden. Die in der zweiten Teilkondensation gebildete zweite Gasfraktion weist insbesondere 59 bis 62 Molprozent Wasserstoff, 9 bis 11 Molprozent Methan, 29 bis 31 Molprozent Kohlenwasserstoffe mit zwei Kohlenstoffatomen und 0,6 bis 0,9 Molprozent Kohlenwasserstoffe mit drei und mehr Kohlenstoffatomen auf. Die in der zweiten Teilkondensation gebildete zweite Flüssigfraktion weist insbesondere 1,5 bis 2 Molprozent Wasserstoff, 3 bis 4 Molprozent Methan, 89 bis 92 Molprozent Kohlenwasserstoffe mit zwei Kohlenstoffatomen und 2 bis 3 Molprozent Kohlenwasserstoffe mit drei und mehr Kohlenstoffatomen auf.

Die erfindungsgemäßen Maßnahme umfassen ferner, dass zumindest ein Teil der ersten Flüssigfraktion und der zweiten Flüssigfraktion von dem ersten Druckniveau auf ein zweites Druckniveau entspannt und unter Erhalt einer dritten Gasfraktion und einer dritten Flüssigfraktion einer Rektifikation unterworfen werden. Für diese Rektifikation kann eine Rektifikationskolonne zum Einsatz kommen, die nachfolgend und insbesondere auch in der Figurenbeschreibung als Deethanisierungskolonne bezeichnet wird. Die Begriffe werden hier synonym verwendet. Im Sumpf einer entsprechenden Rektifikationskolonne werden dabei die Kohlenwasserstoffe mit drei und mehr Kohlenstoffatomen im Wesentlichen abgeschieden, so dass das Kopfgas der Rektifikationskolonne, das zur Bildung der dritten Gasfraktion verwendet wird, im Wesentlichen frei von solchen Komponenten ist.

Die zweite Gasfraktion (nach der zweiten Teilkondensation von dem zweiten auf das dritte Temperaturniveau) und die dritte Gasfraktion (aus der Rektifikation) werden dabei im Rahmen der vorliegenden Erfindung derart gebildet, dass sie mehr als 95%, 96%, 97%, 98% oder 99% Wasserstoff, Methan und Kohlenwasserstoffe mit zwei Kohlenstoffatomen aufweisen. Die dritte Flüssigfraktion wird andererseits im Rahmen der vorliegenden Erfindung derart gebildet, dass sie mehr als 95%, 96%, 97%, 98% oder 99% Kohlenwasserstoffe mit drei und mehr Kohlenstoffatomen aufweist.

Auch im Rahmen herkömmlicher Deethanisierungsschritte werden zwei Gasfraktionen gebildet, die, wie auch im Zusammenhang mit Figur 1 veranschaulicht, aber hier vom Kopf eines C3-Absorbers und vom Kopf einer Deethanisierungskolonne abgezogen werden. Der C3-Absorber wird im Rahmen der vorliegenden Erfindung nicht benöätigt, so dass ein erfindungsgemäßes Verfahren mit deutlich geringerem Aufwand durchgeführt werden kann. Der C3-Absorber wird im Rahmen der vorliegenden Erfindung durch die erste Teilkondensation in Kombination mit der zweiten Teilkondensation der dabei gebildeten Gasfraktion von dem zweiten auf das dritte Temperaturniveau auf dem ersten Druckniveau ersetzt, bei der die durch die zweite Teilkondensation gebildete zweite Flüssigfraktion (anstelle eines Stoffstroms aus einem C3-Absorber, wie in Figur 1 veranschaulicht) in die Rektifikation geführt und dort unter Bildung der dritten Gasfraktion und der dritten Flüssigfraktion getrennt wird. Das erfindungsgemäße Verfahren kann dabei ohne nennenswerte Änderungen in den der Deethanisierung nachgeschalteten Verfahrensschritten implementiert werden, da die für die erste und zweite Teilkondensation verwendeten Wärmetauscher, Kältemittel usw. in einem entsprechenden Verfahren bzw. einer entsprechenden Anlage bereits vorhanden sind und daher weiter verwendet werden können.

In dem erfindungsgemäßen Verfahren liegt das erste Druckniveau insbesondere bei 25 bis 35 bar abs., weiter insbesondere bei 28 bis 30 bar abs., beispielsweise bei ca. 29 bar abs. Das zweite Druckniveau kann im Rahmen der vorliegenden Erfindung insbesondere bei 10 bis 20 bar abs., weiter insbesondere bei 12 bis 16 bar abs., beispielsweise bei ca. 14 bar abs. liegen.

In dem erfindungsgemäßen Verfahren liegt das erste Temperaturniveau insbesondere bei 0 bis 50 °C, weiter insbesondere bei 10 bis 30 °C, beispielsweise bei ca. 20 °C, also im Wesentlichen Umgebungstemperatur. Das zweite Temperaturniveau kann im Rahmen der vorliegenden Erfindung insbesondere bei -30 bis -40 °C, weiter insbesondere bei -33 bis -37 °C, beispielsweise bei ca. -35 °C liegen. Eine entsprechende Abkühlung auf das zweite Temperaturniveau kann insbesondere unter Verwendung eines geeigneten C3-(Propylen-)Kältemittels in einem entsprechenden Wärmetauscher erfolgen. Im Rahmen der vorliegenden Erfindung können zur Abkühlung auf das zweite Temperaturniveau zusätzlich in dem Verfahren oder in nachgeschalteten Verfahren gebildete Stoffströme bzw. Komponentengemische zum Einsatz kommen, beispielsweise ein in dem Verfahren gebildetes Komponentengemisch aus überwiegend oder ausschließlich Wasserstoff und Methan (das nachfolgend als mehr als 95% Wasserstoff und Methan enthaltende Fraktion aus einem weiteren Trennapparat beschrieben wird) und eine in einem nachgeschalteten Trennschritt (C2-Splitter) gebildete Fraktion, die überwiegend oder ausschließlich Ethan aufweist.

Das dritte Temperaturniveau kann im Rahmen der vorliegenden Erfindung insbesondere bei -50 bis -60 °C, weiter insbesonder e bei -52 bis -56 °C, beispielsweise bei ca. -54 °C liegen. Eine entsprec hende Abkühlung auf das dritte Temperaturniveau kann dabei insbesondere unter Verwendung eines geeigneten "Hochdruck"-C2-(Ethylen-)Kältemittels in einem entsprechenden Wärmetauscher erfolgen. Ein entsprechendes Kältemittel liegt dabei insbesondere auf einem Druckniveau von 8 bis 9 bar abs. vor. Im Rahmen der vorliegenden Erfindung können zur Abkühlung auf das dritte Temperaturniveau ebenfalls zusätzlich in dem Verfahren oder in nachgeschalteten Verfahren gebildete Stoffströme bzw. Komponentengemische zum Einsatz kommen, beispielsweise das in dem Verfahren gebildete Komponentengemisch aus überwiegend oder ausschließlich Wasserstoff und Methan und die in dem nachgeschalteten Trennschritt (C2-Splitter) gebildete Fraktion, die überwiegend oder ausschließlich Ethan aufweist. Ferner kann für die Abkühlung auf das dritte Temperaturniveau auch ein in dem Verfahren gebildetes und danach in einen nachgeschalteten Trennschritt (Demethanisierung) geführtes Komponentengemisch, das überwiegend oder ausschließlich Kohlenwasserstoffe mit zwei Kohlenstoffatomen aufweist, verwendet werden. Die Kühlung auf das dritte Temperaturniveau erfolgt insbesondere auch im Gegenstrom zu der bereits mehrfach erwähnten, in der zweiten Teilkondensation gebildeten Flüssigfraktion.

Die dritte Gasfraktion wird im Rahmen der vorliegenden Erfindung in der Rektifikation insbesondere auf einem Temperaturniveau von -25 bis -35 °C, weiter insbesondere von -28 bis -32 °C, beispielsweise ca. -30 °C gebil det. Dies kann insbesondere im Zusammenhang mit einer Kondensation von Kopfgas mit einem geeigneten C3-(Propylen-)Kältemittel erfolgen. Mit anderen Worten wird im Rahmen der vorliegenden Erfindung für die Rektifikation insbesondere eine Rektifikationskolonne verwendet, die mit einem Kopfkondensator gekühlt wird, der mit Propylenkältemittel betrieben wird. Die dritte Flüssigfraktion wird im Rahmen der vorliegenden Erfindung in der Rektifikation vorteilhafterweise auf einem Temperaturniveau von 65 bis 75 °C, weiter insbesondere 68 bis 72 °C, beispielswiese ca. 70 °C, gebildet. Dies lässt sich insbesondere durch die Verwendung eines beispielsweise mit Niederdruckdampf betriebenen Sumpfverdampfers erreichen.

Im Rahmen der vorliegenden Erfindung werden die zweit Gasfraktion und die dritte Gasfraktion, die in den zuvor beschriebenen Verfahrensschritten bereits im Wesentlichen von Kohlenwasserstoffen mit drei und mehr Kohlenstoffatomen befreit wurden, einer nachgeschalteten Demethanisierung zugeführt. Dabei werden insbesondere aus zumindest einem Teil der zweiten Gasfraktion und der dritten Gasfraktion in einem weiteren Trennapparat, nämlich einer Stripkolonne, eine mehr als 95%, 96%, 97%, 98% oder 99% Wasserstoff und Methan enthaltende Fraktion und eine mehr als 95%, 96%, 97%, 98% oder 99% Kohlenwasserstoffe mit zwei Kohlenstoffatomen enthaltende Fraktion gebildet. Auch eine derartige Trennung erfolgt im Rahmen der vorliegenden Erfindung in besonders vorteilhafter Weise, weil hierbei insbesondere ein Trennapparat zum Einsatz kommt, bei dem keine vorstehend beschriebene aufwendige Kondensation von Gas aus dem Trennapparat erfolgen muss. Der Trennapparat in Form einer Stripkolonne wird dabei also ohne Kopfkondensator betrieben. Der weitere Trennapparat wird dabei auf dem zweiten Druckniveau betrieben, wobei ein spezifisch verwendeter Druck auch leicht, d.h. bis zu 1, 2, 3, 4 oder 5 bar unterhalb des Drucks liegen kann, der in der Rektifikation verwendet wird, welcher die erste und zweite Flüssigfraktion zugeführt werden.

Vor der Einspeisung der zweiten Gasfraktion in den weiteren Trennapparat kann dabei insbesondere auch eine stufenweise Abkühlung stehen. In einer besonders bevorzugten Ausführungsform der vorliegenden Erfindung wird dabei zumindest ein Teil der zweiten Gasfraktion mittels einer stufenweisen Abkühlung über ein oder mehrere Zwischentemperaturniveaus auf ein viertes Temperaturniveau auf dem ersten Druckniveau unter Erhalt weiterer Flüssigfraktionen weiteren Teilkondensationen unterworfen. Die jeweils gebildeten Flüssigfraktionen werden dabei vorteilhafterweise entsprechend ihrem jeweiligen Gehalt an Wasserstoff, Methan und Kohlenwasserstoffen mit zwei Kohlenstoffatomen in unterschiedlichen Höhen in den Trennapparat, also die Stripkolonne eingespeist.

Vorteilhafterweise wird ein auf dem vierten Temperaturniveau gasförmig verbleibender Anteil der zweiten Gasfraktion kälteleistend von dem ersten Druckniveau auf das zweite Druckniveau entspannt und in den weiteren Trennapparat eingespeist. Die Einspeisung erfolgt dabei vorteilhafterweise oberhalb jeder der weiteren Flüssigfraktionen, die in den erwähnten weiteren Teilkondensationen über die mehreren Zwischentemperaturen erhalten werden

Im Rahmen der vorliegenden Erfindung liegt das vierte Temperaturniveau insbesondere bei -140 bis -150 °C, weiter insbesond ere bei -140 bis -144 °C, beispielsweise bei ca. -142 °C. Dieses Temperaturni veau lässt sich insbesondere durch einen Stoffstrom erzielen, der aus Kopfgas des Trennapparats gebildet wird. Vorteilhafterweise wird also die mehr als 95%, 96%, 97%, 98% oder 99% Wasserstoff und Methan enthaltende Fraktion dem weiteren Trennapparat entnommen, kälteleistend von dem zweiten Druckniveau auf ein drittes Druckniveau entspannt und zum stufenweisen Abkühlen auf das vierte Temperaturniveau verwendet wird. Die Zwischentemperaturniveaus können dabei insbesondere bei -70 bis - 80 °C, weiter insbesondere bei -76 bis -78 °C, beispielsweise bei ca. -77 °C einerseits und bei -95 bis -105 °C, weiter insbesondere bei -96 bis -100 °C, beispielsweise bei ca. -98 °C andererseits liegen. Diese Temperaturniveaus lassen sich beispielsweise unter Verwendung von "Mitteldruck"-C2-(Ethylen-)Kältemittel auf einem Druckniveau von 3 bis 4 bar einerseits und von "Niederdruck"-C2-(Ethylen-)Kältemittel auf einem Druckniveau von 1,1 bis 1,6 bar andererseits erzielen. In entsprechend verwendeten Wärmetauschern kann insbesondere auch die auf das dritte Druckniveau entspannte und zuvor bereits zur Kühlung auf das vierte Temperaturniveau verwendete, mehr als 95%, 96%, 97%, 98% oder 99% Wasserstoff und Methan enthaltende Fraktion aus dem weiteren Trennapparat verwendet werden.

Mit dem hier beschriebenen Konzept kann die Rückgewinnung der Kohlenwasserstoffe mit zwei Kohlenstoffatomen insbesondere aus dem Spaltgas einer Ethylenanlage mit gasförmigen Einsätzen wie Ethan und Ethan/Propan die komplizierte Deethanisierung und Demethanisierung vereinfacht werden, wobei insbesondere die Deethanisierung ohne C3-Absorber mit aufgesetztem Kopfkondensator erfolgen kann. Die zur Deethanisierung verwendete Rektifikation wird bei einem niedrigen Druck, dem erwähnten zweiten Druckniveau, durchgeführt, so dass sich der Trennaufwand um mehr als 60% reduziert. Folglich kann der Deethanizer bzw. eine entsprechende Rektifikationskolonne viel kleiner ausgebildet werden. Der Sumpf kann mit Waschwasser oder Niederdruckdampf aufgekocht werden. Dies führt zu einem einfacheren Betrieb, geringerem Instrumentierungsaufwand, weniger Fouling sowie geringeren Investitions- und Betriebskosten. Durch den Einsatz der vorliegenden Erfindung kann ferner anstelle einer komplizierten Demethanisierungskolonne eine einfache Stripkolonne eingesetzt werden. Dies führt ebenfalls zu einem einfacheren Betrieb und geringerem Instrumentierungs- und Sicherheitsaufwand. Es ergeben sich geringere Investitionskosten bei gleichbleibendem Energieverbrauch.

In einer besonders bevorzugten Ausgestaltung des erfindungsgemäßen Verfahrens wird der Trennapparat, also die Stripkolonne, mit einem innenliegenden Wärmetauscher ausgestattet, der mit einem Kältemittel auf einem Temperaturniveau von -90 bis -110 °C gekühlt wird. Hierbei kann insb esondere das bereits erläuterte "Niederdruck"-C2-(Ethylen-)Kältemittel eingesetzt werden. Dies ergibt weitere Vorteile, die insbesondere darin bestehen, dass die dritte Gasfraktion aus der Rektifikation zur Deethanisierung auf ein geringeres Temperaturniveau, nämlich nur auf das dritte Temperaturniveau, abgekühlt werden muss, wohingegen bei Fehlen eines entsprechenden innenliegenden Wärmetauscher eine stärkere Abkühlung erfolgen muss. Ferner kann auf eine Unterkühlung eines aus der zweiten Gasfraktion gebildeten Kondensats verzichtet werden.

Die vorliegende Erfindung erstreckt sich auch auf eine Anlage zur Trennung eines Komponentengemischs, das Wasserstoff, Methan, Kohlenwasserstoffe mit zwei Kohlenstoffatomen und Kohlenwasserstoffe mit drei und mehr Kohlenstoffatomen enthält, wobei die Anlage Mittel aufweist, die dafür eingerichtet sind, zunächst wenigstens 95%, 96%, 97%, 98% oder 99% der Kohlenwasserstoffe mit drei und mehr Kohlenstoffatomen aus zumindest einem Teil des Gasgemischs abzutrennen und anschließend aus dem verbleibenden Rest wenigstens 95%, 96%, 97%, 98% oder 99% des Methans und des Wasserstoffs abzutrennen. Die Anlage zeichnet sich durch Mittel aus, die dafür eingerichtet sind, zumindest einen Teil des Komponentengemischs durch Abkühlen von einem ersten Temperaturniveau auf ein zweites Temperaturniveau auf einem ersten Druckniveau unter Erhalt eines ersten Gasfraktion und einer ersten Flüssigfraktion einer ersten Teilkondensation zu unterwerfen, zumindest einen Teil der ersten Gasfraktion durch Abkühlen von dem zweiten Temperaturniveau auf ein drittes Temperaturniveau auf dem ersten Druckniveau unter Erhalt einer zweiten Gasfraktion und einer zweiten Flüssigfraktion einer zweiten Teilkondensation zu unterwerfen, zumindest einen Teil der ersten Flüssigfraktion und der zweiten Flüssigfraktion von dem ersten Druckniveau auf ein zweites Druckniveau zu entspannen und unter Erhalt einer dritten Gasfraktion und einer dritten Flüssigfraktion (C3+) einer Rektifikation zu unterwerfen, die zweite Gasfraktion und die dritte Gasfraktion derart zu bilden, dass sie mehr als 95%, 96%, 97%, 98% oder 99% Wasserstoff, Methan und Kohlenwasserstoffe mit zwei Kohlenstoffatomen aufweisen, und die dritte Flüssigfraktion (C3+) derart zu bilden, dass sie mehr als 95%, 96%, 97%, 98% oder 99% Kohlenwasserstoffe mit drei und mehr Kohlenstoffatomen aufweist.

Eine entsprechende Anlage, die vorteilhafterweise zur Durchführung eines Verfahrens eingerichtet ist, wie es zuvor in unterschiedlichen Ausgestaltungen erläutert wurde, profitiert von den genannten Vorteilen, auf die daher ausdrücklich verwiesen wird.

Die Erfindung wird nachfolgend unter Bezugnahme auf die beigefügten Zeichnungen, die Ausgestaltungen der Erfindung veranschaulichen, weiter erläutert.

Kurze Beschreibung der Zeichnungen
Figur 1 veranschaulicht eine Anlage zur Trennung eines Komponentengemischs.
Figur 2 veranschaulicht eine Anlage zur Trennung eines Komponentengemischs gemäß einer Ausführungsform der Erfindung.
Figur 3 veranschaulicht eine Anlage zur Trennung eines Komponentengemischs gemäß einer Ausführungsform der Erfindung.

In den Figuren sind einander entsprechende Komponenten mit identischen Bezugszeichen angegeben. Auf eine wiederholte Erläuterung entsprechender Komponenten wird der Übersichtlichkeit halber verzichtet. Sämtliche Druck- und Temperaturangaben sind Beispiel- und Ungefährwerte, die in den zuvor ausführlicher erläuterten Bereichen liegen können.

### Ausführliche Beschreibung der Zeichnungen

In Figur 1 ist eine nicht erfindungsgemäße Anlage zur Trennung eines Komponentengemischs in Form eines vereinfachten Prozessflussdiagramms veranschaulicht und insgesamt mit 99 bezeichnet.

Das Komponentengemisch K, beispielsweise ein Spaltgas eines Dampfspaltverfahrens nach Trocknung, Öl- und Benzinentfernung, Sauergasentfernung, Verdichtung und Spaltgashydrierung, wird mit 20°C und 30 bar zunäch st gegen eine Wasserstoff- und Methanfraktion H2/CH4, eine aus einem nicht gezeigten C2-Splitter rückgeführte Ethanfraktion C2REC, ein Kondensat C1 aus einem C3-Absorber T1 und einem C3-Kältemittel C3R in einem Wärmetauscher E1 auf °C abgekühlt und anschließend in einen unteren Teil des C3-Absorbers T1 eingespeist.

Der C3-Absorber T1 ist zweiteilig ausgebildet und verfügt neben dem unteren Teil über einen oberen Teil. Die beiden Teile sind mittels eines Flüssigkeitssperrbodens voneinander getrennt. Auf den oberen Teil wird ein Rücklauf R1 aufgegeben, aus dem unteren Teil das erwähnte Kondensat C1 abgezogen. Das Kondensat C1 wird nach seiner Erwärmung in dem Wärmetauscher E1 in eine Deethanisierungskolonne T2 eingespeist. Ebenso in die Deethanisierungskolonne T2 eingespeist wird eine sich auf dem Flüssigkeitssperrboden des C3-Absorbers T1 ansammelnde Flüssigkeit. Aus dem oberen Teil des C3-Absorbers T1 wird ein Gasstrom G1 abgezogen.

Vom Kopf der Deethanisierungskolonne T2 wird ein Gasstrom G2 abgezogen, in einem Wärmetauscher E7, beispielsweise mittels C3-Kältemittel, abgekühlt und in einem Behälter D1 phasengetrennt. Eine sich in dem Behälter D1 abscheidende Flüssigphase wird über eine Pumpe P1 gefördert und in Form des erwähnten Rücklaufs R1 auf den C3-Absorber T1 und in Form eines weiteren Rücklaufs R2 auf die Deethanisierungskolonne T2 zurückgeführt. Ein in dem Behälter D1 nicht kondensierter Anteil wird in Form eines Gasstroms G3 abgezogen. Sumpfflüssigkeit der Deethanisierungskolonne T2 wird teilweise in einem Wärmetauscher E8, der beispielsweise mittels Niederdruckdampf betrieben wird, verdampft und in die Deethanisierungskolonne T2 zurückgeführt. Weitere Sumpfflüssigkeit wird als überwiegend oder ausschließlich Kohlenwasserstoffe mit drei Kohlenstoffatomen enthaltenden Flüssigstroms C3+ abgezogen.

Die Gasströme G1 und G3, die auf diese Weise im Wesentlichen von Kohlenwasserstoffen mit drei Kohlenstoffatomen befreit sind, werden in einem zweiten Wärmetauscher E2 gegen die Wasserstoff- und Methanfraktion H2/CH4, die aus dem nicht gezeigten C2-Splitter rückgeführte Ethanfraktion C2REC, eine in den C2-Splitter geführte Fraktion C2 und Hochdruck-C2-Kältemittel HP-C2R gekühlt. Der Gasstrom G1 wird auf diese Weise teilkondensiert und zur Phasentrennung in einen Behälter D2 eingespeist. Eine sich in dem Behälter D2 abscheidende Flüssigphase wird in Form eines Flüssigstroms C2 in eine Demethanisierungskolonne T3 eingespeist. Ein in dem Behälter D2 nicht kondensierter Anteil wird als Gasstrom G4 abgezogen.

Der Gasstrom G4 und der ggf. bereits teilkondensierte Gasstrom G3 werden in einem dritten Wärmetauscher E3 gegen die Wasserstoff- und Methanfraktion H2/CH4 und Mitteldruck-C2-Kältemittel MP-C2R gekühlt. Der Gasstrom G4 wird auf diese Weise teilkondensiert und zur Phasentrennung in einen Behälter D3 eingespeist. Eine sich in dem Behälter D3 abscheidende Flüssigphase wird nach Vereinigung mit dem kondensierten Gasstrom G3 als Flüssigstrom C3 in die Demethanisierungskolonne T3 eingespeist. Ein in dem Behälter D3 nicht kondensierter Anteil des Gasstroms G4 wird in Form eines Gasstroms G5 abgezogen.

Der Gasstrom G5 wird in einem vierten Wärmetauscher E4 gegen Niederdruck-C2-Kältemittel LP-C2R gekühlt. Der Gasstrom G5 wird auf diese Weise teilkondensiert und zur Phasentrennung in einen Behälter D4 eingespeist. Eine sich in dem Behälter D4 abscheidende Flüssigphase wird als Flüssigstrom C4 in die Demethanisierungskolonne T3 eingespeist. Ein in dem Behälter D4 nicht kondensierter Anteil des Gasstroms G5 wird als Gasstrom G6 abgezogen. Der Gasstrom G6 wird in einem Expander Ex1 entspannt und in die Demethanisierungskolonne T3 eingespeist.

Die Demethanisierungskolonne T3 ist mehrteilig ausgebildet und umfasst einen unteren, einen mittleren und einen oberen Abschnitt. Vom Kopf der Demethanisierungskolonne T3 bzw. deren oberem Abschnitt wird die Wasserstoff- und Methanfraktion H2/CH4 abgezogen, in einem Expander Ex2 entspannt und zur Kühlung durch Wärmetauscher E5 und E6 geführt. In den Wärmetauschern E5 und E6 werden jeweils Gasströme G7, G8 aus einem oberen Bereich des unteren und mittleren Abschnitts der Demethanisierungskolonne T3 abgezogen, zumindest teilweise kondensiert und als Rücklauf auf die entsprechenden Abschnitte der Demethanisierungskolonne T3 zurückgeführt. Sumpfflüssigkeit der Demethanisierungskolonne T3 wird teilweise in einem Wärmetauscher E9, der beispielsweise mittels Hochdruck-C2-Kältemittel betrieben wird, verdampft und in die Demethanisierungskolonne T3 zurückgeführt. Weitere Sumpfflüssigkeit wird als der Flüssigstrom C2 abgezogen.

Wie erwähnt, sind hier die Komplexität der Deethanisierung mit dem C3-Absorber T1 und die Komplexität der Demethanisierung mit dem Zwischenkühler in Form des Wärmetauschers E6 und den oberhalb der Demethanisierungskolonne angeordneten Wärmetauschern E5 und E6 nachteilig. Letztere erfordern einen erhöhten Instrumentierungs- und Sicherheitsaufwand.

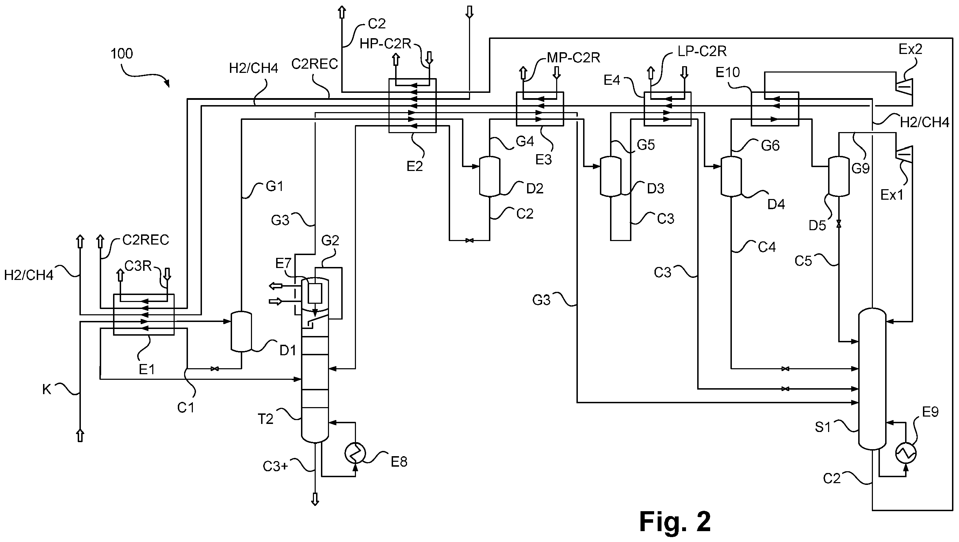

In Figur 2 ist eine Anlage zur Trennung eines Komponentengemischs gemäß einer Ausführungsform der Erfindung in Form eines vereinfachten Prozessflussdiagramms veranschaulicht und insgesamt mit 100 bezeichnet.

Die Abkühlung in dem ersten Wärmetauscher E1 erfolgt wie zur Anlage 99 gemäß Figur 1 erläutert. Stromab des Wärmetauschers E1 ist hier jedoch kein C3-Absorber T1 sondern lediglich ein Behälter D1 vorgesehen, in denen das in dem ersten Wärmetauscher E1 teilkondensierte Komponentengemisch K eingespeist wird. Ein auch hier mit G1 bezeichneter Gasstrom aus dem Behälter D1 weist daher ggf. noch Restgehalte an Kohlenwasserstoffen mit drei Kohlenstoffatomen auf. Diese scheiden sich jedoch nach der weiteren Abkühlung in dem zweiten Wärmetauscher E2, die grundsätzlich identisch wie in dem zweiten Wärmetauscher E2 der Anlage 99 gemäß Figur 1 vorgenommen wird, ab und können in dem auch hier mit C2 bezeichneten Flüssigstrom rückgewonnen werden. Der Flüssigstrom C2 weist ferner vornehmlich Kohlenwasserstoffe mit zwei und drei Kohlenstoffatomen und außerdem geringere Mengen an Wasserstoff und Methan auf.

Der Flüssigstrom C2 wird daher durch den zweiten Wärmetauscher E2 rückgeführt und einer auch hier mit T2 bezeichneten Deethanisierungskolonne zugeführt. Kopfgas von der Deethanisierungskolonne T2 in der Anlage 100 gemäß Figur 2 wird im Gegensatz zu der Deethanisierungskolonne T2 der Anlage 99 gemäß Figur 1 zwar ebenfalls in Form des Gasstroms G2 abgezogen, mittels C3-Kältemittel gekühlt und teilkondensiert; das dabei gebildete Kondensat wird jedoch nur auf die Deethanisierungskolonne T2 selbst und nicht auf einen C3-Absorber zurückgeführt. Ein nicht kondensierter Anteil des Gasstroms G2 wird in Form des Stoffstroms G3, der nun im Wesentlichen frei von Kohlenwasserstoffen mit drei Kohlenstoffatomen ist, abgezogen. Der Stoffstrom G3 wird dabei zunächst im Wesentlichen wie der Stoffstrom G3 in der Anlage 99 gemäß Figur 1 behandelt.

Der Gasstrom G4 und der ggf. bereits teilkondensierte Gasstrom G3 werden auch hier in einem dritten Wärmetauscher E3 gegen die Wasserstoff- und Methanfraktion H2/CH4 und Mitteldruck-C2-Kältemittel MP-C2R gekühlt. Der Gasstrom G4 wird auch hier teilkondensiert und zur Phasentrennung in einen Behälter D3 eingespeist. Eine sich in dem Behälter D3 abscheidende Flüssigphase wird in Form eines Flüssigstroms C3 und ein in dem Behälter D3 nicht kondensierter Anteil des Gasstroms G4 in Form eines Gasstroms G5 abgezogen.

Der Gasstrom G5 und der Flüssigstrom C3 werden hier in einem vierten Wärmetauscher E4 gegen die Wasserstoff- und Methanfraktion H2/CH4 und Niederdruck-C2-Kältemittel LP-C2R gekühlt bzw. unterkühlt. Der Gasstrom G5 wird auf diese Weise teilkondensiert und zur Phasentrennung in einen Behälter D4 eingespeist. Eine sich in dem Behälter D4 abscheidende Flüssigphase wird anschließend als Flüssigstrom C4 in eine Stripkolonne S1 eingespeist, ebenso wie der Flüssigstrom C3. Ein in dem Behälter D4 nicht kondensierter Anteil des Gasstroms G5 wird als Gasstrom G6 abgezogen.

Der Gasstrom G6 wird nun jedoch, im Unterschied zu der Anlage 99 gemäß Figur 1, in einem weiteren Wärmetauscher E10 abgekühlt, der mit entspanntem Kopfgas der Stripkolonne S1, also der Wasserstoff- und Methanfraktion H2/CH4, gekühlt wird. Der auf diese Weise teilkondensierte Gasstrom G6 wird in einem weiteren Behälter D5 phasengetrennt. Erst eine hier verbleibende Gasphase wird über einen Expander, der auch hier mit Ex1 bezeichnet ist, entspannt und in die Stripkolonne S1 eingespeist. Die sich in dem weiteren Behälter D5 abscheidende Flüssigphase wird in Form eines Flüssigstroms C5 ebenfalls in die Stripkolonne S1 eingespeist.

Die Stripkolonne S1 ist einteilig ausgebildet und weist lediglich einen Sumpfverdampfer auf. Vom Kopf der Stripkolonne S1 wird die Wasserstoff- und Methanfraktion H2/CH4 abgezogen, in einem auch hier mit Ex2 bezeichneten Expander entspannt. Die Wärmetauscher E5 und E6 der Anlage 99 gemäß Figur 1 sind nun durch den Wärmetauscher 10 ersetzt. Die Wasserstoff- und Methanfraktion H2/CH4 wird durch die Wärmetauscher E10, E4, E3, E2 und E1 geführt. Sumpfflüssigkeit der Stripkolonne S1 wird teilweise in einem Wärmetauscher E9, der beispielsweise mittels Hochdruck-C2-Kältemittel betrieben wird, verdampft und in die Stripkolonne S1 zurückgeführt. Weitere Sumpfflüssigkeit wird als der Flüssigstrom C2 abgezogen.

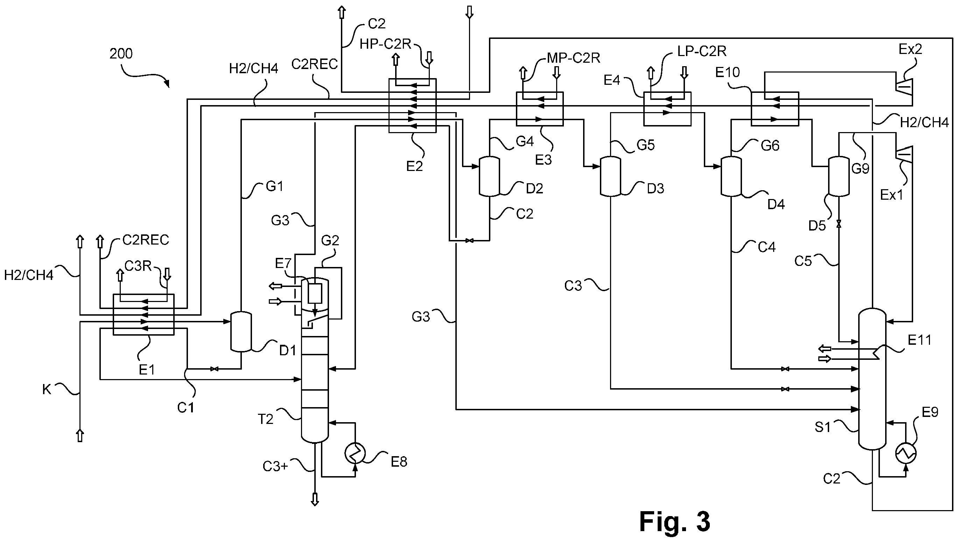

In Figur 3 ist eine Anlage zur Trennung eines Komponentengemischs gemäß einer Ausführungsform der Erfindung in Form eines vereinfachten Prozessflussdiagramms veranschaulicht und insgesamt mit 200 bezeichnet.

Die in Figur 3 veranschaulichte Anlage 200 unterscheidet sich von der in Figur 2 veranschaulichten Anlage 100 insbesondere dadurch, dass ein weiterer, in die Stripkolonne S1 integrierter Wärmetauscher E11 vorhanden ist, der mit Niederdruck-C2-Kältemittel gekühlt wird. Der Wärmetauscher E11 ist dabei zwischen den Einspeisungsstellen der Kondensate C4 und C5 aus den Behältern D4 und D10 vorgesehen. Mit dieser Maßnahme kann das Kopfgas der Deethanisierungskolonne T2, d.h. der Gasstrom G3 nach Abkühlung im zweiten Wärmetauscher E2 direkt in die Stripkolonne S1 eingeleitet werden und das Kondensat aus dem Behälter D3, also der Flüssigstrom C3, muss nicht im vierten Wärmetauscher E4 unterkühlt werden. Infolgedessen wird der Verbrauch an Mitteldruck- und Niederdruck-C2-Kältemittel im dritten Wärmetauscher E3 und im vierten Wärmetauscher E4 stark reduziert und somit der gesamte Verbrauch an C2-Kältemittel.

## Patentansprüche

1. Verfahren zur Trennung eines Komponentengemischs (K), das Wasserstoff, Methan, Kohlenwasserstoffe mit zwei Kohlenstoffatomen und Kohlenwasserstoffe mit drei und mehr Kohlenstoffatomen enthält, wobei
- zunächst wenigstens 95% der Kohlenwasserstoffe mit drei und mehr Kohlenstoffatomen aus zumindest einem Teil des Gasgemischs abgetrennt werden und anschließend aus dem verbleibenden Rest wenigstens 95% des Methans und des Wasserstoffs abgetrennt werden,
**dadurch gekennzeichnet, dass**
- zumindest ein Teil des Komponentengemischs (K) durch Abkühlen von einem ersten Temperaturniveau auf ein zweites Temperaturniveau auf einem ersten Druckniveau unter Erhalt eines ersten Gasfraktion (G1) und einer ersten Flüssigfraktion (C1) einer ersten Teilkondensation unterworfen wird,
- zumindest ein Teil der ersten Gasfraktion (G1) durch Abkühlen von dem zweiten Temperaturniveau auf ein drittes Temperaturniveau auf dem ersten Druckniveau unter Erhalt einer zweiten Gasfraktion (G4) und einer zweiten Flüssigfraktion (C2) einer zweiten Teilkondensation unterworfen wird,
- zumindest ein Teil der ersten Flüssigfraktion (C1) und der zweiten Flüssigfraktion (C1) von dem ersten Druckniveau auf ein zweites Druckniveau entspannt und unter Erhalt einer dritten Gasfraktion (G3) und einer dritten Flüssigfraktion (C3+) einer Rektifikation unterworfen werden,
- die zweite Gasfraktion (G4) und die dritte Gasfraktion (G3) derart gebildet werden, dass sie mehr als 95% Wasserstoff, Methan und Kohlenwasserstoffe mit zwei Kohlenstoffatomen aufweisen, und
- die dritte Flüssigfraktion (C3+) derart gebildet wird, dass sie mehr als 95% Kohlenwasserstoffe mit drei und mehr Kohlenstoffatomen aufweist.

2. Verfahren nach Anspruch 1, bei dem das erste Druckniveau bei 25 bis 35 bar abs. und das zweite Druckniveau bei 10 bis 20 bar liegen.

3. Verfahren nach Anspruch 1 oder 2, bei dem das erste Temperaturniveau bei 0 bis 50 °C, das zweite Temperaturniveau bei -30 bis -40 °C und das dritte Temperaturniveau bei -50 bis -60 °C liegen.

4. Verfahren nach einem der vorstehenden Ansprüche, bei dem die dritte Gasfraktion in der Rektifikation auf einem Temperaturniveau von -25 bis -35 °C gebildet wird und bei dem die dritte Flüssigfraktion in der Rektifikation auf einem Temperaturniveau von 65 bis 75 °C gebildet wird.

5. Verfahren nach einem der vorstehenden Ansprüche, bei dem zur Rektifikation eine Rektifikationskolonne (T2) verwendet wird, die mit einem Kopfkondensator gekühlt wird, der mit Propan- und/oder Propylenkältemittel betrieben wird.

6. Verfahren nach einem der vorstehenden Ansprüche, bei aus zumindest ein Teil der zweiten Gasfraktion (G4) und der dritten Gasfraktion (G3) in einem weiteren Trennapparat (S1) eine mehr als 95% Wasserstoff und Methan enthaltende Fraktion und eine mehr als 95% Kohlenwasserstoffe mit zwei Kohlenstoffatomen enthaltende Fraktion gebildet werden.

7. Verfahren nach Anspruch 6, bei dem der weitere Trennapparat (S1) auf dem zweiten Druckniveau betrieben wird.

8. Verfahren nach Anspruch 6 oder 7, bei dem zumindest ein Teil der zweiten Gasfraktion (G4) mittels einer stufenweisen Abkühlung über ein oder mehrere Zwischentemperaturniveaus auf ein viertes Temperaturniveau auf dem ersten Druckniveau unter Erhalt weiterer Flüssigfraktionen (C3, C4, C5) weiteren Teilkondensationen unterworfen wird.

9. Verfahren nach Anspruch 8, bei dem die weiteren Flüssigfraktionen in den Trennapparat (S1) eingespeist werden.

10. Verfahren nach Anspruch 8 oder 9, bei dem ein auf dem vierten Temperaturniveau gasförmig verbleibender Anteil (G9) der zweiten Gasfraktion (G4) kälteleistend von dem ersten Druckniveau auf das zweite Druckniveau entspannt und in den weiteren Trennapparat (S1) eingespeist wird.

11. Verfahren nach einem der Ansprüche 8 bis 10, bei dem das vierte Temperaturniveau bei -140 bis -150 °C liegt.

12. Verfahren nach einem der Ansprüche 8 bis 11, bei dem die mehr als 95% Wasserstoff und Methan enthaltende Fraktion dem weiteren Trennapparat (S1) entnommen, kälteleistend von dem zweiten Druckniveau auf ein drittes Druckniveau entspannt und zum stufenweisen Abkühlen auf das vierte Temperaturniveau verwendet wird.

13. Verfahren nach einem der Ansprüche 6 bis 12, bei dem der Trennapparat (S1) mit einem innenliegenden Wärmetauscher betrieben wird, der mit einem Kältemittel auf einem Temperaturniveau von -90 bis -110 °C gekü hlt wird.

14. Anlage (100, 200) zur Trennung eines Komponentengemischs (K), das Wasserstoff, Methan, Kohlenwasserstoffe mit zwei Kohlenstoffatomen und Kohlenwasserstoffe mit drei und mehr Kohlenstoffatomen enthält, wobei die Anlage (100, 200) Mittel aufweist, die dafür eingerichtet sind,
- zunächst wenigstens 95% der Kohlenwasserstoffe mit drei und mehr Kohlenstoffatomen aus zumindest einem Teil des Gasgemischs abzutrennen und anschließend aus dem verbleibenden Rest wenigstens 95% des Methans und des Wasserstoffs abzutrennen,
**gekennzeichnet durch** Mittel, die dafür eingerichtet sind,
- zumindest einen Teil des Komponentengemischs (K) durch Abkühlen von einem ersten Temperaturniveau auf ein zweites Temperaturniveau auf einem ersten Druckniveau unter Erhalt eines ersten Gasfraktion (G1) und einer ersten Flüssigfraktion (C1) einer ersten Teilkondensation zu unterwerfen,
- zumindest einen Teil der ersten Gasfraktion (G1) durch Abkühlen von dem zweiten Temperaturniveau auf ein drittes Temperaturniveau auf dem ersten Druckniveau unter Erhalt einer zweiten Gasfraktion (G4) und einer zweiten Flüssigfraktion (C2) einer zweiten Teilkondensation zu unterwerfen,
- zumindest einen Teil der ersten Flüssigfraktion (C1) und der zweiten Flüssigfraktion (C1) von dem ersten Druckniveau auf ein zweites Druckniveau zu entspannen und unter Erhalt einer dritten Gasfraktion (G3) und einer dritten Flüssigfraktion (C3+) einer Rektifikation zu unterwerfen,
- die zweite Gasfraktion (G4) und die dritte Gasfraktion (G3) derart zu bilden, dass sie mehr als 95% Wasserstoff, Methan und Kohlenwasserstoffe mit zwei Kohlenstoffatomen aufweisen, und
- die dritte Flüssigfraktion (C3+) derart zu bilden, dass sie mehr als 95% Kohlenwasserstoffe mit drei und mehr Kohlenstoffatomen aufweist.

15. Anlage (100, 200) nach Anspruch 14, die Mittel aufweist, die zur Durchführung eines Verfahrens nach einem der Ansprüche 1 bis 11 eingerichtet sind.
